# EUROPÄISCHE PATENTANMELDUNG

(11) **EP 4 438 178 A1**
(43) Veröffentlichungstag der Anmeldung: **02.10.2024**
(21) Anmeldenummer: 23166035.8
(22) Anmeldetag: 31.03.2023
(51) Int. Cl.: B01L 3/00, A61B 5/15, G01N 33/53

(54) **BEHÄLTER ZUR AUFNAHME EINER KAPILLARBLUTPROBE UND KIT ZUR GEWINNUNG EINER KAPILLARBLUTPROBE**

(71) Anmelder: MVZ Medizinische Labore Dessau Kassel GmbH, 06847 Dessau (DE)
(72) Erfinder: Böttcher, Michael, 22395 Hamburg (DE)
(74) Vertreter: Zenz Patentanwälte Partnerschaft mbB

(57) **Zusammenfassung**

Die Erfindung betrifft einen Behälter (1) zur Aufnahme einer Kapillarblutprobe mit einem Probenaufnahmeraum (4) zur Aufnahme einer eine vorgegebene Blutmenge enthaltenden Glaskapillare (5) und einem Verschluss (2) zum Verschließen des Probenaufnahmeraums (4). Um eine In-vitro-Bildung von Phosphatidylethanol weiter zu reduzieren und den Anwendungsbereich auf weitere Analyten zu erweitern, enthält der Probenaufnahmeraum (4) erfindungsgemäß eine Mischung (6) einer vorgegebenen Menge Propan-2-ol mit einer vorgegebenen Menge wenigstens einer Säure und/oder eines sauren Salzes der Säure. Die vorgegebene Menge des Propan-2-ols ist so gewählt, dass die Propan-2-ol-Konzentration nach einem Mischen mit der vorgegebenen Blutmenge zwischen 65 Vol.-% und 75 Vol.-%, vorzugsweise zwischen 69 Vol.-% und 71 Vol.-% liegt, besonders bevorzugt 70 Vol.-% beträgt. Die vorgegebene Menge der wenigstens einen Säure und/oder sauren Salzes ist so gewählt, dass sich nach dem Mischen mit der vorgegebenen Blutmenge ein pH-Wert zwischen 5,5 und 6,4, vorzugsweise zwischen 5,7 und 6,1, besonders bevorzugt ein pH-Wert von 5,9, einstellt. Ferner betrifft die Erfindung ein Kit zur Gewinnung von Kapillarblutproben mit wenigstens einem solchen Behälter (1) und wenigstens einer EDTA-beschichteten Glaskapillare (5) zur Aufnahme der vorgegebenen Blutmenge.

## Beschreibung

Die Erfindung betrifft einen Behälter zur Aufnahme einer Kapillarblutprobe, wobei der Behälter einen Probenaufnahmeraum zur Aufnahme einer eine vorgegebene Blutmenge enthaltenden Glaskapillare und einen Verschluss zum dichten Verschließen des Probenaufnahmeraums aufweist. Ferner betrifft die Erfindung ein Kit zur Gewinnung von Kapillarblutproben, aufweisend einen Behälter zur Aufnahme einer Kapillarblutprobe und wenigstens eine EDTA-beschichtete Glaskapillare zur Aufnahme der vorgegebenen Blutmenge.

Derartige Behälter beziehungsweise Kits sind beispielsweise - in der Präanalytik der Bestimmung des Alkoholbiomarkers Phosphatidylethanol - für die Aufnahme und den Transport von Kapillarblutproben zwischen Probennahme und Analytik bekannt (vgl. J. Neumann und M. Böttcher, "Zur Präanalytik der PEth Bestimmung", Zeitschrift für Verkehrssicherheit 5, 2022, S. 377-382). Die bekannten Behälter sind 1,5ml-Schraubdeckelgefäße zur Aufnahme einer mit 20 µl Kapillarvollblut befüllten EDTA-beschichteten Glaskapillare in einem Probenaufnahmeraum, in dem 240 µl Isopropanol (Propan-2-ol) vorgelegt sind. Nach Einbringen der blutgefüllten Kapillare in den Probenaufnahmeraum und dessen Verschließen wird der Behälter sofort geschüttelt. Durch das Propan-2-ol sollen die im Blut enthaltenen Enzyme, insbesondere die Phospholipase D, inaktiviert werden, um eine In-vitro-Bildung von Phosphatidylethanol nach Probennahme zu verhindern.

Aufgabe der Erfindung ist es, die Gefahr einer In-vitro-Bildung von Phosphatidylethanol weiter zu reduzieren und den Anwendungsbereich des Behälters zur Aufnahme der Kapillarblutprobe auf weitere Analyten zu erweitern.

Diese Aufgabe wird erfindungsgemäß durch einen Behälter zur Aufnahme einer Kapillarblutprobe mit den Merkmalen des Anspruchs 1 und durch ein Kit zur Gewinnung von Kapillarblutproben mit den Merkmalen des Anspruchs 7 gelöst.

Erfindungsgemäß ist der eingangs genannte Behälter zur Aufnahme einer Kapillarblutprobe, der einen Probenaufnahmeraum zur Aufnahme einer eine vorgegebene Blutmenge enthaltenden Glaskapillare und einen Verschluss zum dichten Verschließen des Probenaufnahmeraums aufweist, dadurch gekennzeichnet, dass der Probenaufnahmeraum eine Mischung einer vorgegebenen Menge Propan-2-ol mit einer vorgegebenen Menge wenigstens einer Säure und/oder eines sauren Salzes der Säure enthält, wobei die vorgegebene Menge des Propan-2-ols so gewählt ist, dass die Propan-2-ol-Konzentration nach einem Mischen mit der vorgegebenen Blutmenge zwischen 65 Vol.-% und 75 Vol.-%, vorzugsweise zwischen 69 Vol.-% und 71 Vol.-% liegt, besonders bevorzugt 70 Vol.-% beträgt, und wobei die vorgegebene Menge der wenigstens einen Säure und/oder sauren Salzes so gewählt ist, dass sich nach dem Mischen mit der vorgegebenen Blutmenge ein pH-Wert zwischen 5,5 und 6,4, vorzugsweise zwischen 5,7 und 6,1, besonders bevorzugt ein pH-Wert von 5,9, einstellt.

Der Erfindung liegt einerseits die Erkenntnis zugrunde, dass zum Vermeiden einer In-Vitro-Bildung des Alkoholbiomarkers Phosphatidylethanol eine Lyse der Erythrozyten (Hämolyse) erwünscht ist, dass aber die relativ hohe Konzentration des Propan-2-ols bei dem bekannten Verfahren lediglich zu einem Fixieren, aber nicht oder in zu geringem Maße zu einer Hämolyse führt. Es wurde gefunden, dass sich bei einer Konzentration zwischen 65 Vol.-% und 75 Vol.-%, vorzugsweise zwischen 69 Vol.-% und 71 Vol.-% liegt, besonders bevorzugt 70 Vol.-%, die lysierende und zugleich bakterizide Wirkung des Propan-2-ols entfaltet. Darüber hinaus ist Propanol-2-ol preiswert, toxikologisch unbedenklich und umweltfreundlich. Andererseits wurde gefunden, dass bei einem pH-Wert zwischen 5,5 und 6,4, vorzugsweise zwischen 5,7 und 6,1, insbesondere bei einem pH-Wert von 5,9, der Anwendungsbereich des Behälters zur Aufnahme der Kapillarblutprobe auf weitere Analyten erweitert wird, indem diese stabiler in der Probe gehalten werden, d.h. die Gefahr von In-vitro-Veränderungen nach der Probennahme verringert wird. Der erfindungsgemäß angestrebte pH-Wert stellt ein Optimum dar. Einerseits ist er ausreichend sauer (kleiner als 6), um eine erste Gruppe von gewünschten Analyten stabil zu halten. Andererseits weicht er nur so wenig vom ursprünglichen pH-Wert der Blutprobe ab, dass er keine negativen Auswirkungen auf eine zweite Gruppe von Analyten hat, die an sich keiner pH-Wert-Absenkung bedürfen. Beispiele von weiteren Analyten, deren Konzentrationen neben der des Phosphatidylethanol erfindungsgemäß stabiler in der Probe gehalten werden können, sind Zopiclon, Flupirtin, Tilidin sowie Mephedron, Methylen und andere Cathinon-Derivate.

Bei einer Ausführungsform ist der Behälter zur Aufnahme einer Kapillarblutprobe dadurch gekennzeichnet, dass die wenigstens eine Säure eine α-Hydroxycarbonsäure, vorzugsweise eine Säure aus einer Gruppe ist, die Citronensäure, Milchsäure und Weinsäure umfasst. Solche Säuren sind einerseits preiswert und andererseits toxikologisch unbedenklich.

Bei einer weiteren, bevorzugten Ausführungsform ist der Behälter zur Aufnahme einer Kapillarblutprobe dadurch gekennzeichnet, dass das saure Salz ein saures Salz einer α-Hydroxycarbonsäure, vorzugsweise Mononatriumcitrat ist. Vorzugsweise enthält der Probenaufnahmeraum eine Mischung einer vorgegebenen Menge Propan-2-ol mit einer vorgegebenen Menge Mononatriumcitrat. Mononatriumcitrat ist preiswert und toxikologisch unbedenklich (als Lebensmittelzusatzstoff zugelassen - E-Nummer: E 331i).

Bei einer bevorzugten Ausführungsform, bei der die vorgegebene Blutmenge 20 µl beträgt, enthält der Probenaufnahmeraum 240 µl der Mischung der vorgegebenen Menge Propan-2-ol mit der vorgegebenen Menge der wenigstens einen Säure und/oder eines sauren Salzes der Säure. Dies gestattet die Verwendung von EDTA-beschichteten Präzisions-Glaskapillaren mit einem Volumen von 20 µl (Abweichung kleiner als 0,6 %). Bei dieser bevorzugten Ausführungsform enthält die Mischung im Probenaufnahmeraum vorzugsweise eine wässrige Lösung mit 75,8 Vol.-% Propan-2-ol, hergestellt aus deionisiertem Wasser und Propan-2-ol.

Das erfindungsgemäße Kit zur Gewinnung von Kapillarblutproben umfasst wenigstens einen Behälter zur Aufnahme einer Kapillarblutprobe der oben genannten Art und wenigstens eine EDTA-beschichtete Glaskapillare zur Aufnahme der vorgegebenen Blutmenge. Vorzugsweise umfasst das Kit einen Transportbehälter mit mehreren Behältern zur Aufnahme einer Kapillarblutprobe, einen Vorratsbehälter mit EDTA-beschichteten Glaskapillaren und einen Glaskapillarhalter.

Vorteilhafte und oder bevorzugte Weiterbildungen der Erfindung sind in den Unteransprüchen gekennzeichnet.

Nachfolgend wird die Erfindung anhand eines bevorzugten, in der Zeichnung dargestellten Ausführungsbeispiels näher beschrieben, wobei
Fig. 1 einen erfindungsgemäßen Behälter zur Aufnahme einer Kapillarblutprobe und daneben eine Glaskapillare zeigt und
Fig. 2 den erfindungsgemäßen Behälter gemäß Fig. 1, in dessen Probenaufnahmeraum die Glaskapillare aufgenommen ist.

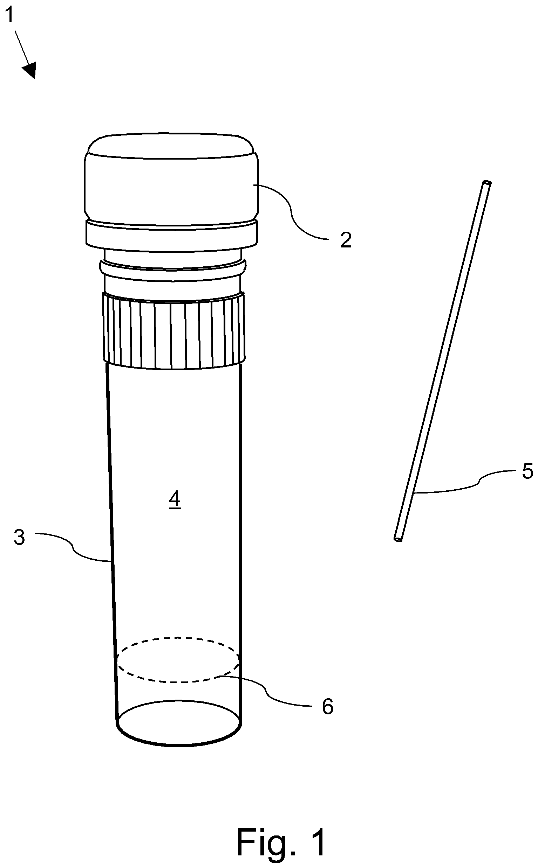

Fig. 1 zeigt links ein Mikroprobengefäß (1) als Behälter zur Aufnahme einer Kapillarblutprobe und rechts eine Glaskapillare (5) zur Aufnahme einer Kapillarblutprobe.

Das Mikroprobengefäß (1) weist ein einen Probenaufnahmeraum (4) umschließendes zylindrisches Aufnahmegefäß (3) und einen Schraubdeckel (2) zum dichten Verschließen des Probenaufnahmeraums (4) auf, die vorzugsweise aus Kunststoff, insbesondere Polypropylen, hergestellt sind.

Bei dem gegenwärtig bevorzugten Ausführungsbeispiel ist in dem Probenaufnahmeraum (4) eine Mischung (6) einer vorgegebenen Menge Propan-2-ol mit einer vorgegebenen Menge Mononatriumcitrat (auch kurz als Citrat bezeichnet) vorgelegt, wobei die vorgegebene Menge des Propan-2-ols so gewählt ist, dass die Propan-2-ol-Konzentration nach einem Mischen mit der in der Glaskapillare (5) aufgenommenen Blutmenge etwa 70 Vol.-% beträgt, und wobei die vorgegebene Menge des Mononatriumcitrats so gewählt ist, dass sich nach dem Mischen mit der Blutmenge ein pH-Wert von etwa 5,9 einstellt.

Die Glaskapillare (5) ist vorzugsweise eine mit Ethylendiamintetraessigsäure (EDTA) beschichtete Präzisions-Glaskapillare mit einem Volumen von 20 µl (Abweichung kleiner als 0,6 %). Bei der für solche Glaskapillaren (5) vorgesehenen Ausführungsform werden im Probenaufnahmeraum (4) von 2ml-Mikroprobengefäßen jeweils 240 µl der Propan-2-ol-Citrat-Wasser-Mischung (6) mit 75,8 Vol.-% Propan-2-ol, hergestellt aus Mononatriumcitrat, deionisiertem Wasser und Propan-2-ol, vorgelegt.

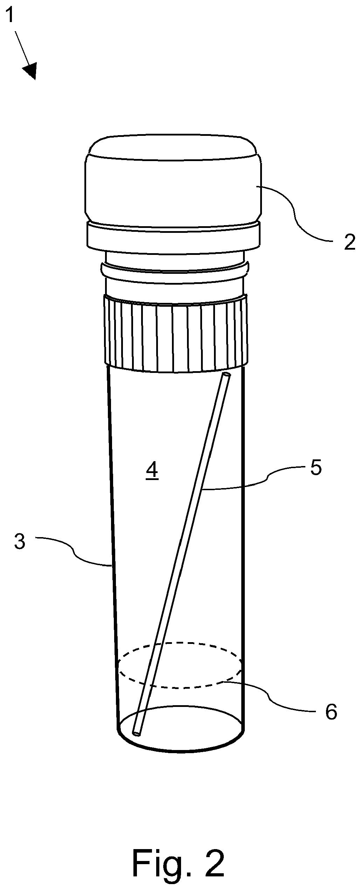

Fig. 2 zeigt schematisch den Behälter gemäß Fig. 1, in dessen Probenaufnahmeraum (4) die Glaskapillare (5) aufgenommen ist. Bei der Probennahme wird die blasenfrei blutgefüllte Glaskapillare (5) in den Probenaufnahmeraum (4) gebracht, der Behälter dicht verschlossen und dann derart geschüttelt, dass sich das Blut aus der Glaskapillare (5) mit der vorgelegten Propan-2-ol-Mononatriumcitrat-Wasser-Mischung vermischt.

Eine Charge der erfindungsgemäßen Mikroprobengefäße (1), in denen eine vorgegebene Menge einer Mischung (6) aus Propan-2-ol, deionisiertem Wasser und Mononatriumcitrat vorgelegt ist, wird beispielsweise wie folgt hergestellt.

Eine Charge umfasst beispielsweise 1000 Mikroprobengefäße. Für eine solche Charge werden zunächst 250 ml der Propan-2-ol-Citrat-Wasser-Mischung mit einem Propan-2-ol-Gehalt von 75.8% frisch hergestellt. Dazu werden in eine Vorratsflasche 147 mg Natriummonocitrat mit der Feinwaage eingewogen. Anschließend werden mit einem Messzylinder 60 ml deionisiertes Wasser zugegeben und so lange geschüttelt, bis der Feststoff vollständig gelöst ist. Danach werden 190 ml Propan-2-ol (hoher Reinheit, beispielsweise "LC-MS grade", wobei "LC-MS" von "Liquid Chromatography - Mass Spectrometry" herrührt) zugegeben und erneut gut homogenisiert. Dann werden 1000 Mikroprobengefäße (2 ml, mit Verschluss) bereitgestellt. Für die Verteilung der in den Mikroprobengefäßen vorzulegenden Mischungsmengen von jeweils 240 µl wird vorzugsweise ein entsprechend programmierter Pipettierroboter verwendet. Die befüllten Mikroprobengefäße werden mit den Schraubverschlüssen dicht verschlossen und in Transportboxen zur Aufnahme von beispielsweise jeweils 50 Mikroprobengefäßen einsortiert.

Ein Kit zur Gewinnung von Kapillarblutproben umfasst beispielsweise eine Transportbox mit 50 Mikroprobengefäßen, die jeweils 240 µl der Propan-2-ol-Citrat-Wasser-Mischung enthalten, einen Behälter mit 100 Stück EDTA-beschichteter 20 µl-Glaskapillaren, einen Glaskapillarhalter, einen Behälter mit Desinfektionsmittel, eine sterile Sicherheits-Einweg-Lanzette mit Klinge und eine entsprechende Anzahl von Barcode-Etiketten zur Aufbringung auf die mit Blutproben befüllten Mikroprobengefäße. Das Kit kann auch als Kapillarblutentnahmesystem bezeichnet werden.

Das erfindungsgemäße Kapillarblutentnahmesystem eignet sich nicht nur für eine verbesserte Präanalytik für die Alkoholbiomarker Phosphatidylethanole (PEth), sondern stellt auch eine Verbesserung der Präanalytik für eine Vielzahl weiterer Analyten dar, insbesondere für missbrauchsrelevante Drogen. Beispiele von weiteren Analyten sind der Alkoholbiomarker Ethylglucuronid (EtG), Cannabinoide, Zopiclon, Flupirtin, Tilidin sowie Mephedron, Methylen und andere Cathinon-Derivate. Eine erste Gruppe der Analyten sind im schwach Alkalischen nicht stabil. Es wurde gefunden, dass diese im Sauren für eine längere Zeit stabil bleiben. Der pH-Wert von etwa 5,9 ist ausreichend sauer, um diese erste Gruppe von gewünschten Analyten stabil zu halten. Andererseits weicht er nur so wenig vom ursprünglichen pH-Wert der Blutprobe ab, dass er keine negativen Auswirkungen auf eine zweite Gruppe von Analyten hat, die an sich keiner pH-Wert-Absenkung bedürfen.

## Patentansprüche

1. Behälter (1) zur Aufnahme einer Kapillarblutprobe,
wobei der Behälter (1) einen Probenaufnahmeraum (4) zur Aufnahme einer eine vorgegebene Blutmenge enthaltenden Glaskapillare (5) und einen Verschluss (2) zum dichten Verschließen des Probenaufnahmeraums (4) aufweist,
**dadurch gekennzeichnet,**
**dass** der Probenaufnahmeraum (4) eine Mischung (6) einer vorgegebenen Menge Propan-2-ol mit einer vorgegebenen Menge wenigstens einer Säure und/oder eines sauren Salzes der Säure enthält,
wobei die vorgegebene Menge des Propan-2-ols so gewählt ist, dass die Propan-2-ol-Konzentration nach einem Mischen mit der vorgegebenen Blutmenge zwischen 65 Vol.-% und 75 Vol.-%, vorzugsweise zwischen 69 Vol.-% und 71 Vol.-% liegt, besonders bevorzugt 70 Vol.-% beträgt, und
wobei die vorgegebene Menge der wenigstens einen Säure und/oder sauren Salzes so gewählt ist, dass sich nach dem Mischen mit der vorgegebenen Blutmenge ein pH-Wert zwischen 5,5 und 6,4, vorzugsweise zwischen 5,7 und 6,1, besonders bevorzugt ein pH-Wert von 5,9, einstellt.

2. Behälter (1) zur Aufnahme einer Kapillarblutprobe nach Anspruch 1, **dadurch gekennzeichnet, dass** die wenigstens eine Säure eine α-Hydroxycarbonsäure, vorzugsweise eine Säure aus einer Gruppe ist, die Citronensäure, Milchsäure und Weinsäure umfasst.

3. Behälter (1) zur Aufnahme einer Kapillarblutprobe nach Anspruch 1 oder 2, **dadurch gekennzeichnet, dass** das saure Salz ein saures Salz einer α-Hydroxycarbonsäure, vorzugsweise Mononatriumcitrat ist.

4. Behälter (1) zur Aufnahme einer Kapillarblutprobe nach Anspruch 1, **dadurch gekennzeichnet, dass** der Probenaufnahmeraum (4) eine Mischung (6) einer vorgegebenen Menge Propan-2-ol mit einer vorgegebenen Menge Mononatriumcitrat enthält.

5. Behälter (1) zur Aufnahme einer Kapillarblutprobe nach einem der Ansprüche 1 - 4, **dadurch gekennzeichnet, dass** die vorgegebene Blutmenge 20 µl beträgt und dass der Probenaufnahmeraum (4) 240 µl der Mischung (6) der vorgegebenen Menge Propan-2-ol mit der vorgegebenen Menge der wenigstens einen Säure und/oder eines sauren Salzes der Säure enthält.

6. Behälter (1) zur Aufnahme einer Kapillarblutprobe nach Anspruch 5, **dadurch gekennzeichnet, dass** die Mischung (6) im Probenaufnahmeraum (4) 75,8 Vol.-% Propan-2-ol enthält.

7. Kit zur Gewinnung von Kapillarblutproben, umfassend
wenigstens einen Behälter (1) zur Aufnahme einer Kapillarblutprobe nach einem der Ansprüche 1 - 6 und
wenigstens eine EDTA-beschichtete Glaskapillare (5) zur Aufnahme der vorgegebenen Blutmenge.

8. Kit nach Anspruch 7, umfassend einen Transportbehälter mit mehreren Behältern (1) zur Aufnahme einer Kapillarblutprobe, einen Vorratsbehälter mit EDTA-beschichteten Glaskapillaren (5) und einen Glaskapillarhalter.
